# EUROPÄISCHE PATENTSCHRIFT

(11) **EP 2 822 520 B1**
(45) Veröffentlichungstag und Bekanntmachung des Hinweises auf die Patenterteilung: **28.10.2015**
(21) Anmeldenummer: 13710313.1
(22) Anmeldetag: 05.03.2013
(51) Int. Cl.: A61F 5/01, B29C 41/20, A61F 13/08, A61F 13/02, A61F 13/10, A61F 5/40, B29K 305/00, B29L 31/00, B29K 83/00

(54) **VERFAHREN ZUR HERSTELLUNG VON BANDAGEN**
METHOD FOR PRODUCING BANDAGES
PROCÉDÉ DE FABRICATION DE BANDAGES

(30) Priorität: 08.03.2012 DE 102012101937
(43) Veröffentlichungstag der Anmeldung: 14.01.2015
(73) Patentinhaber: NTT New Textile Technologies GmbH, 72336 Balingen (DE)
(72) Erfinder: BAUER, Hans, 72336 Balingen (DE)
(74) Vertreter: Müller, Gottfried
(86) Internationale Anmeldenummer: PCT/EP2013/054342
(87) Internationale Veröffentlichungsnummer: WO 2013/131878

(56) Entgegenhaltungen:
- EP-A1- 0 027 172
- EP-A1- 0 496 071
- EP-A2- 1 086 669
- WO-A1-2011/007179

## Beschreibung

Die Erfindung bezieht sich auf ein Verfahren zur Herstellung von Bandagen.

Bekannt sind Gelenkbandagen aus einem elastischen Stoffmaterial, auf das ein vorgefertigtes Silikon-Formteil durch Kleben, Schweißen oder Aufnähen aufgebracht ist. Das Formteil sorgt für eine zusätzliche Stabilität und eine bessere Abstützung des Gelenks, um das die Bandage nach Art einer Manschette geschlungen wird. Das Formteil wird als vorgefertigtes Bauteil, beispielsweise in Ringform, auf die elastische Stofflage aufgebracht und mit der Stofflage verbunden. Das Formteil kann von einer zweiten Stofflage überspannt sein.

Zur Herstellung derartiger Bandagen sind mehrere Verfahrensschritte erforderlich. Zunächst muss das Formteil aus Silikon hergestellt werden, anschließend wird das Formteil in geeigneter Weise mit der Stofflage verbunden und schließlich von einer weiteren Stofflage überspannt.

Aus der DE 601 04 663 T2 ist es bekannt, einen orthopädischen Gegenstand wie z.B. eine orthopädische Sohle unter Verwendung eines Blocks aus Silikonelastomer, auf den ein textiles Material aufgebracht wird, herzustellen. Hierbei wird zunächst eine Seite des textilen Materials mit einem Zweikomponenten-Silikonkleber beschichtet und anschließend die beschichtete Seite des textilen Materials auf den vorgefertigten Block aus Silikonelastomer aufgelegt. Anschließend wird der Verbund von textilem Material und Block aus Silikonelastomer für eine definierte Zeitspanne auf eine erhöhte Temperatur erhitzt, um eine Vernetzung des Silikonklebers zu erzielen.

Die EP 0 496 071 A1 offenbart eine Gelenkbandage mit einem elastomeren Formteil, das auf eine elastische Stofflage aufgebracht ist und über eine Stoffseite übersteht.

Auch die EP 0 027 172 A1 offenbart eine Gelenkbandage, die aus einem elastischen Bandagenstoff besteht, in den Kompressionseinlagen eingebracht sind.

Die EP 1 086 669 A2 offenbart ein orthopädisches Polster mit einer weichelastischen Einlage, die durch Inkontaktbringen und Verfestigung eines fließfähigen Materials am Polster erzeugt wird.

Der Erfindung liegt die Aufgabe zugrunde, in flexibel handhabbarer und einfach durchzuführender Weise eine Bandage herzustellen, die mit einem stützenden Formteil versehen ist.

Diese Aufgabe wird erfindungsgemäß mit den Merkmalen des Anspruches 1 gelöst. Die Unteransprüche geben zweckmäßige Weiterbildungen an.

Das erfindungsgemäße Verfahren bezieht sich auf die Herstellung von Bandagen, beispielsweise Gelenkbandagen wie Knie- oder Ellbogenbandagen, bei dem eine elastische Stofflage mit einem elastischen und dreidimensional geformten Formteil versehen wird, welches der Bandage zusätzliche Stabilität verleiht und das Körperteil, um das die Bandage geschlungen wird, in gewünschter Weise stützt. Es wird eine bessere Abstützung der Gelenke, von Sehnen oder Muskelpartien erreicht. Das Formteil besteht aus einem Elastomer, das im nicht abgebundenen, zähflüssigen oder zumindest formbaren Zustand auf die Stofflage aufgebracht wird. Das nicht abgebundene Elastomer kann in die Stofflage eindringen und sich beim Aushärten mit der Stofflage verbinden, so dass keine weiteren Maßnahmen zum Verbinden des Formteils mit der Stofflage erforderlich sind. Allein durch das Aufbringen des Elastomers im nicht abgebundenen Zustand auf die Stofflage und dem anschließenden Aushärten wird eine ausreichende, feste Verbindung zwischen der Stofflage und dem Formteil aus dem Elastomer geschaffen.

Gegenüber Ausführungen aus dem Stand der Technik können mehrere Arbeitsschritte beim Herstellungsprozess eingespart werden. Mit dem Aufbringen des Elastomers auf die Stofflage wird in einem gemeinsamen Arbeitsschritt das Formteil erzeugt und zugleich mit der Stofflage verbunden. Zusätzliche Maßnahmen zum Verbinden des Formteils mit der Stofflage sind nicht erforderlich, es wird insbesondere kein Kleber zum Verbinden des Elastomer-Formteils mit der Stofflage benötigt. Das Elastomer ist im nicht abgebundenen Zustand, in welchem der Auftrag auf die Stofflage erfolgt, hinreichend formbar, so dass das Formteil in der gewünschten Form erzeugt werden kann. Zugleich ist das Elastomer zähflüssig genug, um ein Fließen zu verhindern bzw. auf ein Minimum zu beschränken, so dass die gewünschte dreidimensionale Form erzeugt werden kann. Im abgebundenen Zustand weist das Formteil, welches aus dem Elastomer besteht, eine ausreichend hohe Eigenelastizität auf, so dass gemeinsam mit der elastischen Stofflage eine Bandage mit der geforderten Elastizität entsteht.

Das Elastomer, aus dem das Formteil gebildet wird, kann als ein stark haftendes Silikon ausgeführt sein, das beim Anlegen der Bandage unmittelbar in Hautkontakt gelangt und die Wirkung einer medizinischen Wundversorgung entfaltet. Durch das Anhaften auf der Haut ist zugleich die Positionierung der Bandage gewährleistet.

Das Auftragen des Elastomers erfolgt bevorzugt im Wege des Spritzverfahrens. Hierbei wird das Elastomer in seinem nicht abgebundenen Zustand auf die Stofflage aufgespritzt und härtet auf der Stofflage aus, wodurch das dreidimensionale Formteil gebildet wird. Im nicht abgebundenen Zustand weist das Elastomer eine ausreichend hohe Fließfähigkeit auf, die ein Aufspritzen auf die Stofflage erlaubt.

Das Aufspritzen erfolgt vorzugsweise über eine Düse, was den Vorteil hat, dass das Elastomer mit hoher Präzision auf die Stofflage aufgebracht werden kann.

Gemäß einer weiteren zweckmäßigen Ausführung wird das Elastomer schichtweise aufgetragen. Nach dem Aufbringen einer Schicht bzw. Lage härtet das Elastomer zumindest teilweise aus und erlangt dadurch eine höhere Festigkeit, woraufhin die nächste Lage bzw. Schicht von Elastomeren aufgetragen werden kann. Auf diese Weise wird das Elastomer Schicht für Schicht aufgebracht, insbesondere aufgespritzt, bis die gewünschte dreidimensionale Form des Formteils erreicht wird. Nach dem Aufbringen jeder Schicht härtet das Elastomer zumindest teilweise so weit aus, bis das Aufbringen der nächsten Schicht möglich ist.

Gemäß einer weiteren vorteilhaften Ausführung wird das Elastomer unmittelbar auf die Stofflage aufgebracht bzw. aufgespritzt, wobei auf den Einsatz eines Klebers zum Verbinden mit der Stofflage verzichtet werden kann.

Es kann zweckmäßig sein, das Elastomer nach dem Aufbringen auf die Stofflage mit einem Beflockungsmaterial zu beschichten, um den Tragekomfort zu erhöhen. Das Beflockungsmaterial kann auf das noch nicht vollständig ausgehärtete Elastomer aufgebracht werden, so dass das Beflockungsmaterial mit dem Aushärten fest an dem Formteil anhaftet. Ein Kleber für das Verbinden des Beflockungsmaterials mit dem Elastomer ist nicht erforderlich. Grundsätzlich möglich ist aber auch ein nachträgliches Aufbringen des Beflockungsmaterials auf das bereits ausgehärtete Formteil, wobei in diesem Fall zweckmäßigerweise ein Kleber eingesetzt wird, damit das Beflockungsmaterial am Formteil anhaftet. Als Beflockungsmaterial kommen Naturfasern, synthetische Fasern oder halbsynthetische Fasern in Betracht.

Das Elastomer wird vorzugsweise nur an einer Seite auf die Stofflage aufgebracht, so dass sich das aus Elastomer bestehende Formteil auch nur über diese Seite über die Stofflage dreidimensional erhebt. Die Beflockung erfolgt vorzugsweise auch nur an der Seite, an der das Formteil übersteht. Möglich ist aber auch eine Beflockung auf der Gegenseite, soweit das Elastomer in nicht abgebundenem Zustand durch die Stofflage hindurchdringt.

Gemäß einer weiteren zweckmäßigen Ausführung ist vorgesehen, dass auf beide Seiten der Stofflage ein Elastomer zur Erzeugung eines Formteils aufgebracht wird, wobei die Formteile entweder spiegelsymmetrisch zueinander angeordnet sind oder eine voneinander abweichende Position und/oder Geometrie aufweisen.

Es kann zweckmäßig sein, in das Elastomer ein Verstärkungselement einzubetten, welches die Funktion hat, dem Formteil eine höhere Stabilität zu verleihen. Des Weiteren erlaubt das Verstärkungselement einen dreidimensionalen Höhenaufbau des Formteils, der höher sein kann und in beliebiger Weise geformt sein kann als dies ausschließlich über eine Elastomerraupe im nicht abgebunden Zustand realisierbar ist. Das Verstärkungselement wird vorteilhafterweise von dem Elastomer umspritzt.

Als Verstärkungselement kommt beispielsweise ein Schaumstoffteil in Betracht, das bereits die gewünschte Grundform aufweist, welche auch das Elastomer-Formteil einnimmt. Das Schaumstoffteil wird mit Elastomer im nicht abgebundenen Zustand bedeckt, anschließend härtet das Elastomer aus, wobei durch die Eigenelastizität des Elastomers sowie des Schaumstoffes auch das Formteil, bestehend aus Schaumstoffteil und Elastomer, die gewünschten elastischen Eigenschaften aufweist.

Als Verstärkungselement kommt auch ein Bauteil aus Metall, Kunststoff, Keramik oder Ähnlichem in Betracht, das zusätzlich zur Formgebungsfunktion auch eine Verstärkungsfunktion hat. So ist es beispielsweise möglich, als Verstärkungselement Federn in das Elastomer einzubetten, die dem Formteil und der Stofflage eine gewünschte Federwirkung aufprägen.

Als Elastomer wird beispielsweise ein Silikon verwendet, das im nicht abgebundenen Zustand zähflüssig ist und beispielsweise über eine Düse auf die Stofflage bzw. das Verstärkungselement, welches auf der Stofflage aufliegt, aufgebracht werden kann. Die Düse hat den Vorteil, dass das Elastomer mit hoher Präzision auf die Stofflage aufgebracht werden kann. Auch der schichtweise Aufbau des Formteils mit verschiedenen aufeinanderliegenden Elastomerschichten bzw. -lagen kann präzise mithilfe der Düse durchgeführt werden.

Das Verstärkungselement wird zweckmäßigerweise nicht unmittelbar mit der Stofflage verbunden. Die Befestigung des Verstärkungselementes erfolgt vorzugsweise ausschließlich über das Elastomer, in das das Verstärkungselement eingebettet ist. Ein Ankleben des Verstärkungselements auf die Stofflage ist nicht erforderlich.

Grundsätzlich kommen verschiedene Möglichkeiten in Betracht, das Elastomer bzw. Formteil auf der Stofflage anzuordnen. Möglich ist es beispielsweise, das Formteil mit Abstand zum Rand der Stofflage zu positionieren, wobei grundsätzlich auch Ausführungen möglich sind, bei denen das Formteil sich bis zum Randbereich der Stofflage erstreckt.

Weitere Vorteile und zweckmäßige Ausführungen sind den weiteren Ansprüchen, der Figurenbeschreibung und den Zeichnungen zu entnehmen. Es zeigen:
- Fig. 1: in Draufsicht eine Bandage, die eine Stofflage aufweist, auf die elastische Formteile aus Silikon aufgebracht sind,
- Fig.2: die Bandage gemäß Fig. 1 im Schnitt,
- Fig. 3: ein ringförmiges Verstärkungselement aus Schaumstoff, welches zum Herstellen der Bandage in Silikon eingebettet wird,
- Fig. 4: ein stabförmiges Federelement aus Metall, das als Verstärkungselement ebenfalls in Silikon auf der Stofflage eingebettet werden kann.

In den Figuren sind gleiche Bauteile mit gleichen Bezugszeichen versehen.

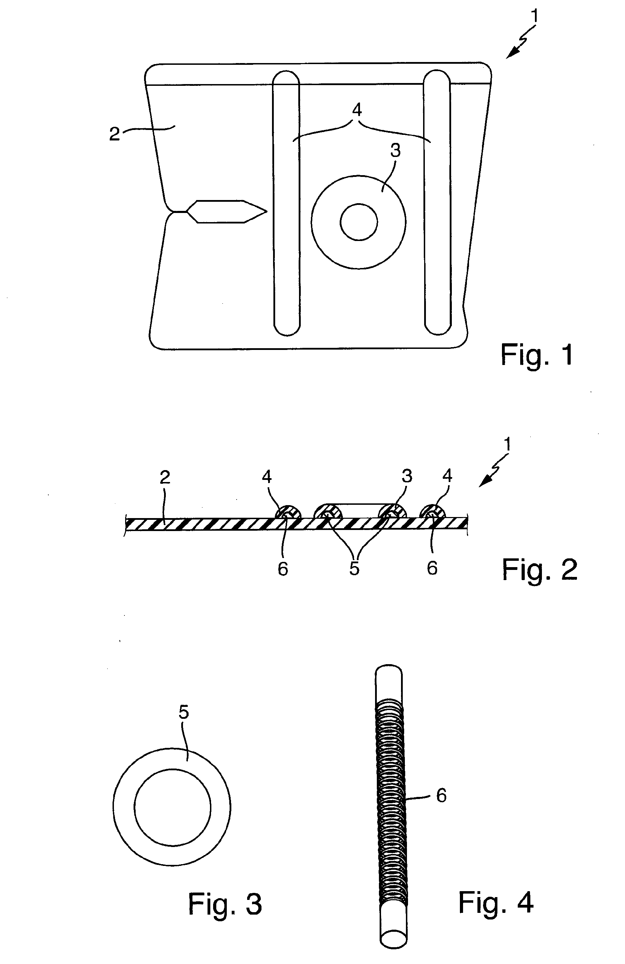

In den Fig. 1 und 2 ist eine Gelenkbandage 1 in Draufsicht bzw. im Schnitt dargestellt. Bei der Gelenkbandage 1 handelt es sich zum Beispiel um eine Kniebandage oder um eine Ellbogenbandage, die zur Stützung des Gelenks um die betreffende Gliedmaße gewickelt und fixiert wird. Die Bandage 1 weist eine elastische Stofflage 2 auf, auf die Formteile 3 und 4 aufgebracht sind, welche die Aufgabe haben, die Bandage zu verstärken und im ungebundenen Zustand das Gelenk zu stützen. Das erste Formteil 3 ist ringförmig ausgebildet, die beiden Formteile 4 an unterschiedlichen Seiten des Ringes 3 sind stabförmig ausgeführt.

Die Formteile 3 und 4 bestehen jeweils aus einem Elastomer wie zum Beispiel Silikon, in das ein Verstärkungselement 5, 6 eingebettet ist. Das Elastomer wird im fließfähigen, nicht abgebundenen Zustand auf die Stofflage 2 aufgebracht, wobei die Verstärkungselemente 5, 6 auf der Stofflage aufliegen und von dem Elastomermaterial umspritzt werden. Nach dem Aushärten bzw. Abbinden des Elastomers sind die Verstärkungselemente 5, 6 vollständig von dem Elastomer eingehüllt. Beim Herstellungsprozess dringt Elastomermaterial in die Stofflage 2 ein und sorgt beim Aushärten für eine Verbindung des Formteils 3 mit der Stofflage 2. Da die Verstärkungselemente 5, 6 von dem Elastomer 3, 4 eingehüllt sind, sind auch die Verstärkungselemente sicher an der Stofflage 2 gehalten.

Wie der Schnittdarstellung gemäß Fig. 2 zu entnehmen, handelt es sich bei den Formteilen 3, 4 um dreidimensional geformte, elastische Polster, die über eine Seite der Stofflage 2 überstehen. Die Formgebung wird wesentlich durch die Verstärkungselemente 5, 6 erreicht. Da die Verstärkungselemente 5, 6 vollständig von dem Elastomermaterial eingehüllt sind, ist die Gesamthöhe und -breite der Formteile 3 größer als diejenige der Verstärkungselemente 5, 6.

Wie den Fig. 3 und 4 zu entnehmen, handelt es sich bei dem Verstärkungselement 5 um ein ringförmiges Schaumstoffteil und bei dem Verstärkungselement 6 um ein stabförmiges Metall-Federelement, welches auf Biegung beanspruchbar ist. Das Schaumstoffteil 6 ist elastisch nachgiebig ausgeführt. Aufgrund der Elastizität bzw. Biegsamkeit der Verstärkungselemente 5, 6 sowie der Eigenelastizität des abgebundenen, ausgehärteten Elastomermaterials und der Elastizität der Stofflage 2 ist sichergestellt, dass die Bandage 1 über eine verhältnismäßig hohe Elastizität verfügt bei zugleich großer Stabilität. Über die Verstärkungselemente ist außerdem eine gezielte Richtungstabilität einstellbar, beispielsweise dergestalt, dass das stabförmige Federelement 6 nur um seine Querachse biegbar ist, jedoch in Richtung der Längsachse keine relevante Elastizität aufweist.

Die Verstärkungselemente 5, 6 liegen während des Herstellungsprozesses nur lose auf der Stofflage 2 auf, die Verbindung zur Stofflage erfolgt über das Formteil 3 mit dem Elastomermaterial, welches in die Stofflage 2 eindringt und aushärtet.

Die Formteile 3, 4 werden zweckmäßigerweise mit einem Beflockungsmaterial beschichtet, um den Tragekomfort zu erhöhen. Bei dem Beflockungsmaterial handelt es sich um organische, synthetische oder halbsynthetische Fasern, die entweder vor dem Aushärten des Elastomermaterials aufgebracht werden, so dass das Beflockungsmaterial eine feste Verbindung mit dem Elastomermaterial eingeht. Möglich ist es aber auch, erst nach dem Aushärten des Elastomermaterials das Beflockungsmaterial aufzubringen, wobei in diesem Fall ein zusätzlicher Klebstoff eingesetzt wird.

Zum Herstellen der Bandage werden zunächst die Formteile 3 und 4 auf die Stofflage 2 aufgesetzt. Anschließend wird nicht abgebundenes Elastomermaterial wie zum Beispiel Silikon über eine oder mehrere Düsen in der Weise auf die Stofflage 2 aufgetragen, dass die Verstärkungsteile 5, 6 vollständig in das Elastomermaterial eingebettet werden. Danach kann das Elastomermaterial aushärten, wobei gegebenenfalls vor dem Erreichen des Aushärtens Beflockungsmaterial aufgebracht wird.

## Patentansprüche

1. Verfahren zur Herstellung von Bandagen (1), insbesondere Gelenkbandagen wie zum Beispiel Knie- oder Ellbogenbandagen, bei dem ein Elastomer im noch nicht abgebundenen Zustand auf eine elastische Stofflage (2) schichtweise aufgetragen wird, wobei das Elastomer nach dem Abbinden ein mit der Stofflage (2) verbundenes, elastisches und dreidimensionales Formteil (3, 4) bildet, das über mindestens eine Stoffseite übersteht.

2. Verfahren nach Anspruch 1,
**dadurch gekennzeichnet,**
**dass** das Elastomer nach dem Aufbringen mit einem Beflockungsmaterial beschichtet wird.

3. Verfahren nach Anspruch 2,
**dadurch gekennzeichnet,**
**dass** das Beflockungsmaterial auf das nicht-abgebundene Elastomer aufgebracht wird.

4. Verfahren nach einem der Ansprüche 1 bis 3,
**dadurch gekennzeichnet,**
**dass** als Elastomer Silikon verwendet wird.

5. Verfahren nach einem der Ansprüche 1 bis 4,
**dadurch gekennzeichnet,**
**dass** in das Elastomer ein Verstärkungselement (5, 6) eingebettet wird.

6. Verfahren nach Anspruch 5,
**dadurch gekennzeichnet,**
**dass** das Verstärkungselement (5, 6) auf die Stofflage (2) aufgelegt und anschließend das Elastomer um das Verstärkungselement (5, 6) gespritzt wird.

7. Verfahren nach einem der Ansprüche 1 bis 6,
**dadurch gekennzeichnet,**
**dass** das Elastomer mit Abstand zum Rand der Stofflage (2) aufgebracht wird.

8. Verfahren nach einem der Ansprüche 1 bis 7,
**dadurch gekennzeichnet,**
**dass** das Elastomer ohne Kleber auf die Stofflage aufgebracht wird.

## Claims

1. Method for producing bandages (1), in particular joint bandages, for example knee bandages or elbow bandages, in which method an elastomer, in the as yet uncured state, is applied in a layered formation onto an elastic fabric layer (2), wherein the elastomer, after curing, forms an elastic and three-dimensional shaped part (3, 4) which is connected to the fabric layer (2) and which projects from at least one side of the fabric.

2. Method according to Claim 1, **characterized in that** the elastomer, after it has been applied, is coated with a flocking material.

3. Method according to Claim 2, **characterized in that** the flocking material is applied to the uncured elastomer.

4. Method according to one of Claims 1 to 3, **characterized in that** silicone is used as elastomer.

5. Method according to one of Claims 1 to 4, **characterized in that** a reinforcement element (5, 6) is embedded into the elastomer.

6. Method according to Claim 5, **characterized in that** the reinforcement element (5, 6) is laid onto the fabric layer (2), and the elastomer is then injected around the reinforcement element (5, 6).

7. Method according to one of Claims 1 to 6, **characterized in that** the elastomer is applied at a distance from the edge of the fabric layer (2).

8. Method according to one of Claims 1 to 7, **characterized in that** the elastomer is applied without adhesive to the fabric layer.

## Revendications

1. Procédé de fabrication de bandages (1), en particulier de bandages d'articulations, par exemple des bandages de genou ou de coude, dans lequel un élastomère à l'état non encore réticulé est appliqué en couche sur une couche (2) de matière élastique, l'élastomère formant après sa réticulation une pièce façonnée (3, 4) reliée à la couche de matière (2), élastique et tridimensionnelle, qui déborde d'au moins un côté de la matière.

2. Procédé selon la revendication 1, **caractérisé en ce que** l'élastomère est revêtu d'un matériau de flocage après son application.

3. Procédé selon la revendication 2, **caractérisé en ce que** le matériau de flocage est appliqué sur l'élastomère non réticulé.

4. Procédé selon l'une des revendications 1 à 3, **caractérisé en ce qu'**il utilise comme élastomère un silicone.

5. Procédé selon l'une des revendications 1 à 4, **caractérisé en ce qu'**un élément de renfort (5, 6) est incorporé dans l'élastomère.

6. Procédé selon la revendication 5, **caractérisé en ce que** l'élément de renfort (5, 6) est placé sous la couche de matière (2), l'élastomère étant ensuite projeté autour de l'élément de renfort (5, 6).

7. Procédé selon l'une des revendications 1 à 6, **caractérisé en ce que** l'élastomère est appliqué à distance du bord de la couche de matière (2).

8. Procédé selon l'une des revendications 1 à 7, **caractérisé en ce que** l'élastomère est appliqué sans adhésif sur la couche de matière.
